# FASCICULE DE BREVET EUROPEEN

(11) **EP 2 877 445 B1**
(45) Date de publication et mention de la délivrance du brevet: **06.09.2017**
(21) Numéro de dépôt: 13756595.8
(22) Date de dépôt: 24.07.2013
(51) Int. Cl.: C09D 4/00, C07C 235/06

(54) **DIAMIDES D'ACIDES GRAS COMPRENANT DES HYDROXYACIDES STEARIQUES COMME ORGANOGELATEURS**
FETTSÄUREDIAMIDE MIT STEARINHYDROXYSÄUREN ALS ORGANOGELATOREN
FATTY ACID DIAMIDES COMPRISING STEARIC HYDROXYACIDS AS ORGANOGELATORS

(30) Priorité: 27.07.2012 FR 1257299
(43) Date de publication de la demande: 03.06.2015
(73) Titulaire: ARKEMA FRANCE, 92700 Colombes (FR)
(72) Inventeur: BERNARD, Michael Y., F-95880 Enghien-les-Bains (FR); DUQUENNE, Christophe, F-75010 Paris (FR); MONNIER, Guillaume, F-60190 Avrigny (FR)
(74) Mandataire: Killis, Andréas
(86) Numéro de dépôt international: PCT/FR2013/051785
(87) Numéro de publication internationale: WO 2014/016517

(56) Documents cités:
- EP-A1- 2 098 502
- FR-A1- 2 281 162
- US-A- 3 977 894
- US-A- 4 128 436
- US-A1- 2006 047 046
- V. AJAY MALLIA ET AL: "Robust Organogels from Nitrogen-Containing Derivatives of ( R )-12-Hydroxystearic Acid as Gelators: Comparisons with Gels from Stearic Acid Derivatives +", LANGMUIR, vol. 25, no. 15, 4 août 2009 (2009-08-04), pages 8615-8625, XP055054611, ISSN: 0743-7463, DOI: 10.1021/la8042439

## Description

L'invention concerne d'abord un diamide d'acide gras comprenant dans sa structure des hydroxyacides carboxyliques spécifiques et l'utilisation de ce produit comme organogélateur ou agent de rhéologie, appelé aussi additif de rhéologie, en particulier dans des compositions de revêtements, de colles ou d'adhésifs, de moulage, de mastics ou d'agents d'étanchéité ou de cosmétique.

Les amides d'acides gras et en particulier les diamides à base d'acide hydroxy-12-stéarique sont déjà connus comme agents organogélateurs, c'est-à-dire des petites molécules organiques capables de gélifier toutes sortes de solvants organiques même à des concentrations massiques relativement faibles (moins de 1% en masse) ou comme additifs de rhéologie, c'est-à-dire permettant de modifier la rhéologie d'une formulation d'application. Ils permettent d'obtenir, par exemple, un effet thixotropique ou pseudoplastique.

US 4 128 436 décrit un agent de contrôle de rhéologie qui comprend un mélange sous forme particulaire d'huile de ricin hydrogéné avec un oligomère polyamide à base d'acide hydroxystéarique, de diamines primaires α-ω saturées et d'acides dicarboxyliques α-ω saturés ou de dimères d'acides hydrogénés.

US 3 977 894 décrit un additif rhéologique pour systèmes non aqueux à base d'argiles montmorillonites modifiées organiques et autoactivées par le fait que lesdites argiles sont dans le mélange homogène avec deux cires solides, l'une à base de triglycéride de 12-HSA et l'autre à base d'un amide à base d'une diamine primaire et de 12-HSA.

US 2006/0047046 décrit l'utilisation d'un agent thixotrope pour composition adhésive anaérobie, cet agent étant basé sur un agent minéral modifié par des sels organiques ou à base d'un agent organique éventuellement combiné avec un agent particulaire minéral non modifié ou un modifié par des sels organiques. Comme agents organiques, sont cités des cires ou des huiles de ricin hydrogénées ou des triglycérides de 12-HSA, éventuellement en présence d'un amide gras d'acide en C₁₀ à C₂₄ afin d'améliorer la dispersibilité des triglycérides.

FR 2 281 162 décrit la solidification ou gélification de liquides organiques non polaires tels que les hydrocarbures en utilisant comme agent de solidification ou de gélification des esters, des amides ou des sels d'amine d'aminoacides, tous N-acylés.

EP 2 098 502 décrit des composés alkyl amides en C₃ à C₂₀₀ ou des diamides comprenant une structure cyclique en C₅ à C₈, ces dernières étant obtenues à partir d'une diamine ou d'un diacide ou d'un aminoacide de structure cyclique en C₅ à C₈ et respectivement d'au moins deux monoacides ou deux monoamines identiques ou différents ou d'un monoacide et d'une monoamine dans le cas d'aminoacide, de longueur identique ou différente, monoamines ou monoacides étant en C₃ à C₂₀₀. Un parmi la liste des nombreux monoacides cités est le 12-HSA. Ces alkyls amides ou ces diamides sont préconisées pour l'utilisation dans les encres pour imprimantes à jet d'encre avec comme effet des améliorations en termes de viscosité d'homogénéité et de coût.

V.A. Mallia et al décrivent dans « Langmuir 2009, 25 (15) 8615-8625 » des dérivés azotés comme amides ou amines, de 12-HSA et d'acide stéarique et comparent leurs propriétés d'organogélateur dans divers solvants.

L'acide hydroxy-12 stéarique noté aussi 12HSA ou 12-HSA pour la suite, est donc couramment utilisé comme matière première pour la préparation d'amides gras. Cependant, cet hydroxy acide carboxylique est issu d'une filière dont la source unique est l'huile de ricin. En raison du développement rapide de certaines applications utilisant largement l'huile de ricin directement ou sous forme de ses dérivés, sa consommation a considérablement augmenté générant des problèmes de disponibilité et de tension sur les prix de ces matières premières issues de la filière huile de ricin, comme le 12-HSA. Il y a donc un besoin croissant de trouver une solution alternative au 12-HSA en cherchant de nouvelles matières premières issues d'une filière indépendante de l'huile de ricin, qui soit à la fois abondante et également d'origine renouvelable (ou biosourcée), pouvant remplacer partiellement, en particulier au moins 10% et de préférence totalement, c'est-à-dire à 100%, le 12-HSA, tout en gardant des performances d'organogélateur ou rhéologiques très satisfaisantes pour diverses applications à des taux en poids inférieurs à 5%, en particulier ne dépassant pas 1% pour une application revêtements.

Aucun des documents cités de l'état de l'art ne décrit ou ne suggère la solution d'un tel problème technique ni ne décrit ni ne suggère la solution de la présente invention.

La présente invention vise des nouveaux diamides gras ayant un taux réduit et de préférence nul, d'acide hydroxy-12 stéarique (12-HSA), lequel est obtenu à partir de l'huile ricinoléïque comme matière de départ et couramment utilisé comme composant de diamides gras. Ceci est réalisé en le remplaçant partiellement, en particulier au moins 20% et de préférence totalement, c'est-à-dire à 100%, par d'autres hydroxyacides carboxyliques gras saturés de source différente de celle de l'huile de ricin, cette source pouvant être celle de l'huile oléique. En effet, l'hydroxylation de l'acide oléique permet d'accéder aux acides hydroxy-9 (noté aussi 9-hydroxy) et hydroxy-10 (noté aussi 10-hydroxy) stéariques respectivement notés 9-HSA et 10-HSA. Ce remplacement est visé de sorte à avoir des performances d'agent organogélateur en milieu solvant organique, tout à fait satisfaisantes en termes de pouvoir thixotrope, en particulier pour des taux en poids inférieurs à 5%, plus particulièrement inférieurs à 1% en poids dans l'application visée et pouvant, dans certains cas, être au moins aussi bonnes que celles des organogélateurs de l'état de la technique, en particulier à base exclusive de 12-HSA. Le remplacement partiel ou total ne doit donc pas affecter les performances rhéologiques des produits diamides ainsi obtenus tout en respectant un environnement durable avec des matières premières d'origine renouvelable.

Dans ce but, le premier objet de la présente invention vise d'une manière générale un diamide d'acide gras dans lequel l'acide 12-HSA est remplacé partiellement et plus particulièrement et de préférence totalement par un autre acide carboxylique gras saturé à base d'acide stéarique ayant un groupement hydroxy en position 9 ou 10, d'origine renouvelable et leurs mélanges respectifs.

Un autre objet de l'invention est un additif, en particulier de rhéologie, comprenant un diamide de l'invention, ledit additif existant ou utilisé sous forme de pâte préactivée et préconcentrée dans un plastifiant ou solvant organique polaire.

L'invention concerne également un organogélateur et plus particulièrement un agent de rhéologie comprenant ledit diamide et l'utilisation dudit diamide comme tel.

Finalement, l'invention couvre une composition de liant organique comprenant en tant qu'agent de rhéologie au moins un diamide selon la présente invention.

Donc, le premier objet de l'invention est un diamide d'acide gras qui comprend au moins un produit de réaction d'un mélange comprenant ou consistant de :
a) au moins une diamine sélectionnée parmi :
   ▪ une diamine aliphatique linéaire en C₂ à C₁₂, de préférence C₂ à C₈ et plus préférentiellement C₂ à C₆, et/ou
   ▪ une diamine cycloaliphatique en C₆ à C₁₈, de préférence C₆ à C₁₂, et/ou
   ▪ une diamine aromatique de préférence en C₆ à C₁₂, plus préférentiellement la xylylène diamine et/ou phénylène diamine,
b) au moins un hydroxyacide carboxylique gras saturé sélectionné parmi l'acide hydroxy-9 stéarique (9-HSA) et/ou l'acide hydroxy-10 stéarique (10-HSA) en présence ou en l'absence d'acide hydroxy-12 stéarique (12-HSA),
c) en option, au moins un monoacide, sélectionné parmi les acides carboxyliques linéaires saturés et non hydroxylés en C₆ à C₁₈, de préférence de C₆ à C₁₅, plus préférentiellement de C₆ à C₁₂
d) en option, au moins une deuxième diamine différente de a) et sélectionnée parmi les amines aliphatiques linéaires en C₂ à C₁₂, de préférence C₂ à C₈ et plus préférentiellement de C₂ à C₆.

Selon un cas plus particulier de l'invention, ledit acide hydroxy-12 stéarique (12-HSA) est présent et le taux des autres acides hydroxy stéariques, c'est-à-dire d'acide hydroxy-9 stéarique et/ou d'acide hydroxy-10 stéarique, varie de 10 à 99%, de préférence de 20 à 99%, plus préférentiellement de 30 à 99% en poids du composant b).

Plus particulièrement, dans ledit diamide de l'invention, ledit composant b) est un mélange d'acide hydroxy-9 stéarique et/ou d'acide hydroxy-10 stéarique et d'acide hydroxy-12 stéarique, avec un rapport molaire de 9-HSA et/ou 10-HSA sur 12-HSA allant de 10/90 à 99/1, plus préférentiellement de 20/80 à 99/1 et plus particulièrement de 30/70 à 99/1.

Selon une autre option préférée, dans ledit diamide, l'acide hydroxy-12 stéarique est absent et il est remplacé totalement, c'est-à-dire à 100%, par l'acide hydroxy-9 stéarique et/ou l'acide hydroxy-10 stéarique. Le remplacement de 12-HSA, qu'il soit partiel ou total, est réalisé de préférence par un mélange de 9-HSA et de 10-HSA, tel qu'obtenu sans séparation particulière à partir de l'acide oléique après hydroxylation de l'acide oléïque, porteur d'une insaturation éthylénique en position 9, laquelle insaturation est saturée par ladite hydroxylation avec obtention dudit mélange en 9-HSA et 10-HSA. Ce mélange pourrait être considéré comme étant équimolaire.

Selon un cas particulier, ledit diamide porte 2 fonctions amides à base du même hydroxyacide b) ou à base de deux hydroxyacides b) différents.

Selon une autre option préférée de l'invention, ledit monoacide c) est présent à un taux tel que le rapport molaire dudit hydroxyacide b) sur ledit monoacide c) est de 1/2 à 4/1. Plus particulièrement encore, ledit diamide de l'invention porte une fonction amide à base d'un hydroxyacide b) et une autre fonction à base dudit monoacide c), ce qui signifie que le rapport molaire b/c est de 1/1.

D'une manière générale, le rapport molaire amine/acide peut varier de 0,9 à 1,1 et de préférence correspond au rapport stoechiométrique de 1/1.

Selon une autre possibilité, ledit diamide selon l'invention comprend au moins 2, de préférence au moins 3 produits de réaction différents, tels que définis ci-haut. Ainsi, ledit diamide peut être un mélange binaire ou ternaire, quaternaire ou plus, de produits différents de réaction comme décrits ci-dessus. Selon un cas plus particulier, selon cette possibilité, ledit diamide de l'invention peut comprendre un mélange de produits comprenant des produits des formules suivantes :
- b1-a1-c1
- b1-a1-b1
- b1-a1-b2
et de préférence :
- b1-a1-c1
- b1-a1-b1
- b1-a1-b2
- b2-a1-c1
- b2-a1-b2
avec a1 : résidu de diamine selon a), b1 : résidu de 9-HSA, b2 : résidu de 10-HSA et c1 : résidu de monoacide non hydroxylé selon monoacide c).

Comme exemple de diamines aliphatiques linéaires convenables et préférées pour le composant diamine a) dudit diamide, on peut citer : l'éthylène diamine, la propylène diamine, la butylène (ou tetraméthylène) diamine, la pentaméthylène diamine, l'hexaméthylène diamine et de préférence l'éthylène diamine ou l'hexaméthylène diamine.

Comme exemple de diamines cycloaliphatiques convenables toujours selon le composant a), on peut citer: la diamine-1,3, -1,4 et -1,2 et en particulier -1,3 ou -1,4 cyclohexane, l'isophorone diamine, la bis (aminométhyl)-1,3, -1,4 ou -1,2 cyclohexane (dérivé de l'hydrogénation de respectivement m-, p-, o- xylylène diamine), de préférence la bis (aminométhyl)-1,3 ou -1,4 cyclohexane, décahydronaphtalène diamine, le bis(3-méthyl, bis (amino-4 cyclohexyl) méthane (BMACM) ou bis (amino-4 cyclohexyl) méthane (BACM), 1-{[4-(aminomethyl)cyclohexyl]oxy}propan-2-amine. Les diamines cycloaliphatiques préférées sont parmi: diamine-1,3, -1,4 cyclohexane, bis (aminométhyl)-1,3, -1,4 ou -1,2 cyclohexane, isophorone diamine, bis (amino-4 cyclohexyl) méthane.

Comme exemple convenable et préféré de diamines aromatiques convenables en tant que composant a) dudit diamide, on peut citer : la m-, p-, o- xylylène diamine, en particulier m- et p-, la m-, p-, o- phénylène diamine, en particulier m- et p- xylylène diamine.

Comme exemple de monoacides c), on peut citer : l'acide hexanoïque, heptanoïque, octanoïque, nonanoïque, décanoïque, undécanoïque ou dodécanoïque (ou laurique) ou stéarique. Sont préférés : hexanoïque, octanoïque, nonanoïque, décanoïque.

Les hydroxyacides b) pouvant remplacer l'acide hydroxy-12 stéarique comme déjà précisé ci-haut sont : l'acide hydroxy-9 et/ou hydroxy-10 stéarique, ces deux derniers pouvant être surtout sous forme de mélange après hydroxylation de l'acide oléïque, lequel porte une insaturation éthylénique en position 9, laquelle insaturation est saturée par l'hydroxylation.

Ledit diamide selon l'invention est utilisé de préférence sous forme de poudre micronisé et plus préférentiellement ayant une taille moyenne en volume inférieure à 50 µ, encore plus préférentiellement inférieure à 25 µ. Plus particulièrement, concernant la distribution, 90% de particules ont une taille inférieure à 20 µ. Ladite taille peut être déterminée par diffraction laser.

Le deuxième objet de l'invention concerne un additif, en particulier additif de rhéologie, lequel comprend au moins un diamide (c'est-à-dire un ou plusieurs) tel que défini selon l'invention ci-haut et lequel existe et est utilisé sous forme de pâte préactivée et préconcentrée dans un solvant organique, de préférence polaire et plus particulièrement ledit additif consiste d'un ou plusieurs diamides tels que définis ci-haut selon l'invention et d'un solvant organique, de préférence polaire, plus préférentiellement avec un taux en poids dudit ou desdits diamides allant de 15 à 45%.

Un autre objet de l'invention concerne l'utilisation du diamide de l'invention en tant qu'organogélateur dans un solvant organique, de préférence solvant organique polaire pour l'obtention d'un organogel, de préférence en tant qu'agent ou additif de rhéologie et plus préférentiellement dans une composition préconcentrée dans un solvant organique, de préférence solvant organique polaire, sous forme de pâte préactivée.

Le terme « solvant organique polaire » selon la description ci-haut, inclut dans sa définition ou est à interpréter comme signifiant « au moins un solvant organique polaire » ou « un mélange de solvants organiques comprenant au moins un solvant organique polaire ». Est considéré comme solvant organique polaire, un solvant comprenant au moins un groupement polaire, comme par exemple groupement alcool ou ester. Comme exemple de solvant organique polaire selon la première option d'interprétation, on peut citer un alcool tel que l'éthanol ou le butanol ou leur mélange et selon la deuxième option d'interprétation (mélange de solvants organiques comprenant au moins un solvant organique polaire) un mélange d'un tel alcool (éthanol ou butanol) avec un solvant non polaire comme par exemple le xylène.

Plus particulièrement ladite utilisation est dans des compositions de revêtements, en particulier peintures, vernis, encres, gel coats ou dans des compositions de colles ou d'adhésifs, de moulage, de mastics ou d'agents d'étanchéité ou d'agent décapant ou de cosmétique.

Est également couvert un organogélateur, de préférence agent ou additif de rhéologie, qui comprend au moins un diamide tel que défini ci-haut selon l'invention. Plus particulièrement, ledit organogélateur est un agent ou additif de rhéologie, en particulier thixotrope, dans une composition préconcentrée dans un solvant organique, de préférence polaire sous forme de pâte préactivée. La préparation de telles pâtes préactivées préconcentrées peut se faire selon la description et exemples de WO 2008/0153924 en remplaçant le plastifiant décrit par un solvant organique, de préférence solvant organique polaire comme défini ci-haut.

Finalement, l'invention couvre une composition de liant organique, laquelle comprend en tant qu'agent de rhéologie au moins un diamide tel que défini selon la présente invention. Plus particulièrement, ledit liant organique est un liant pour compositions de revêtements parmi peintures, vernis, encres, gel coats ou un liant pour compositions de colles ou d'adhésifs ou un liant pour compositions de mastic ou d'agents d'étanchéité ou d'agent décapant ou pour des compositions de moulage ou enfin pour des compositions de cosmétique. De préférence, ledit liant est sélectionné parmi les résines époxy, vinyl esters, polyesters insaturés et saturés, alkydes, résines silanées, polyuréthanes, polyesters-amides, résines acryliques solvantées avec solvant non réactif, monomères et/ou oligomères acrylates multifonctionnels (MFA) ou résines acryliques acrylées avec diluant réactif ou élastomères chlorés ou non chlorés ou autres polymères chlorés.

Les compositions de moulage sont plus particulièrement des compositions de moulage pour composites, en particulier renforcés avec des fibres de renfort, telles que fibres de verre ou de carbone ou d'aramide ou pour pièces moulées, type SMC ou BMC ou stratifiées, types coques de bateaux ou panneaux de composites ou pièces moulées par coulée, avec application de la composition par projection au pistolet ou à la brosse ou au rouleau.

Ces additifs spécifiques permettent de modifier la viscosité des compositions de mastics, de colles, d'adhésifs, de revêtements, tels que les peintures, vernis, gel coats, encres ou de compositions de moulage ou de mastics ou d'agent d'étanchéité ou d'agent décapant ou de cosmétique.

Pour être utilisé comme additif organogélateur et plus particulièrement thixotrope dans une composition d'application telle que de revêtement comme peinture, vernis, gel coat, encre ou une composition de colle ou d'adhésif ou dans une composition d'agent d'étanchéité ou d'agent décapant ou des mastics ou une composition de moulage ou de cosmétique, ledit diamide a besoin d'être activé pour avoir son caractère thixotrope. Selon une première option préférée, ceci peut se faire indépendamment de la composition finale d'application, dans une composition préconcentrée dudit diamide dans un solvant organique, de préférence solvant organique polaire, qui est de préférence liquide à l'ambiante et adapté au diamide et à l'application finale et préactivée sous forme de pâte préactivée, comme décrit dans la description et en particulier dans les exemples de WO 2008/0153924. Dans ce cas, cette composition préactivée de diamide est rajoutée dans la composition d'application finale sans besoin d'activation dans la mesure où ledit diamide est additionné déjà préactivé avec sa composition de pâte « préactivée », « préconcentrée» et adaptée pour l'application finale. Dans ce cas, l'utilisateur final qui est le formulateur, n'aura pas besoin d'activer sa formulation dans la mesure où ledit diamide préactivé ainsi additionné, confère ce caractère dès son mélange dans ladite composition finale d'application, sans besoin d'activation supplémentaire et permettant ainsi une productivité améliorée (réduction du temps de préparation qui devient ainsi plus pratique et simple).

A défaut de préactivation sous forme de pâte préactivée préconcentrée dans un milieu adapté (compatible) à l'application finale, l'activation dudit diamide peut être réalisée selon une deuxième option, in situ dans la composition d'application finale mais par l'utilisateur final. Le diamide de l'invention peut donc être préactivé sous forme de pâte préactivée et préconcentrée.

Cette activation requiert un cisaillement à haute vitesse et un chauffage correspondant avec des montées de température pouvant aller jusqu'à près de 120°C selon les produits, ainsi qu'une durée minimale nécessaire, dépendante des conditions de température et du système, pour développer des propriétés rhéologiques finales optimales. Ces additifs confèrent à la composition à laquelle ils sont incorporés un comportement thixotrope caractérisé par une rhéofluidification marquée, c'est-à-dire une réduction de la viscosité lorsque le cisaillement augmente, puis une reprise en viscosité dépendante du temps (équivalent à un effet d'hystérésis). Ainsi, ce type d'additifs apporte à la composition finale d'excellentes propriétés d'application qui se caractérisent par une forte viscosité au repos, une bonne stabilité de cette viscosité au stockage, une bonne anti-sédimentation, une facilité d'application et d'extrusion suivant l'application et une bonne résistance à la coulure une fois appliquée.

Un diamide d'acide gras de l'invention peut être obtenu par condensation entre au moins une diamine primaire selon a), un hydroxy acide carboxylique gras saturé selon b) et en option en présence d'un acide monocarboxylique selon c), éventuellement en présence d'une deuxième diamine primaire selon d). Le produit de réaction peut optionnellement être dilué dans de l'huile de ricin hydrogénée ou optionnellement dans l'huile oléique hydroxylée et, dans ce cas, à un taux variant de 10 à 100% en poids par rapport au total diamide + huile (par exemple de ricin hydrogénée) et de préférence à un taux variant de 20 à 100% en poids. L'huile de ricin hydrogénée peut être utilisée pour adapter l'affinité du mélange final (diamide + huile de ricin hydrogénée) par rapport à la composition de la formulation finale en application.

Dans le cas de dilution dans l'huile de ricin hydrogénée, l'addition se fait à une température comprise entre 140 et 220°C. A la fin de l'addition, on obtient une masse solide qui est broyée sous forme de poudre.

Ledit diamide d'acide gras peut donc être utilisé sous forme de poudre ou de pâte préactivée comme décrit ci-haut.

Les exemples décrits ci-dessous en partie expérimentale sont présentés pour illustrer l'invention et ses performances et ne limitent en rien la portée revendiquée.

### Partie Expérimentale

### I - Matières premières utilisées

**Tableau 1 : Matières premières utilisées**

| **Produit** | **Fonction** | **Référence commerciale** | **Fournisseur** |
|---|---|---|---|
| Acide Oléique | Réactif | Acide Oléique purifié | VWR |
| Acide Formique | Réactif | Acide Formique, 96% | Aldrich |
| Acide Perchlorique | Réactif | Acide Perchlorique, 70% | Aldrich |
| soude | Réactif | NaOH 6N | VWR |
| Acide chlorhydrique | Réactif | HCl 37% | VWR |
| Hexamethylenediamine | diamine selon a) | Hexamethylenediamine 98% | Aldrich |
| Acide hexanoïque | acide selon c) | Hexanoic acid 99% | Aldrich |
| Ethylene diamine | diamine selon a) | Ethylenediamine ≥ 99.5% (GC) | Aldrich |
| Résine époxy | liant | Araldite^{®} GZ 7071X75 | Huntsman |
| Résine époxy | liant | Araldite^{®} GY 783 BD | Huntsman |
| Agent débullant | Agent débullant | BYK^{®} A530 | Byk |
| Agent Dispersant | Dispersant | Disperbyk^{®} 110 | Byk |
| Dioxide de titane | Pigment | Tiona 595 | Société des ocres de France |
| Oxide de fer | Pigment | Bayferrox^{®} 915 | Lubrizol |
| Phosphate de zinc | Pigment | ZP 10 | HEUCOPHOS |
| Talc | Additif | Finntalc MO5 | Mondo minerals |
| Silice | charge | HPF6 | Sibelco |
| n-Butanol | Solvant | n-Butanol | Aldrich |
| polyamide | Durcisseur | CRAYAMID^{®} 140 | Arkema |
| Xylène | solvant | Xylène, reagent grade | Aldrich |
| Acide 12-hydroxy stéarique | Hydroxy acide selon b) | 12-HSA | JAYANT AGRO |

### II - Méthodes et tests utilisés

Les formulations sont évaluées avec deux tests : le test de résistance à l'écoulement et une évaluation de la viscosité à différentes vitesses.

### - Test de résistance à l'écoulement

Il s'effectue à l'aide d'un contrôleur de coulure (Levelling/Sagging Tester de Sheen Instruments^{®}) qui permet d'établir la résistance d'un revêtement à la coulure due à la gravité. Fabriqué en inox et doté d'une lame droite, ce contrôleur comporte des encoches de valeur croissante.

Le test consiste à déposer différentes bandes parallèles de peinture d'épaisseurs différentes sur une bande de contraste grâce au contrôleur de coulure. La carte de contraste est immédiatement placée en position verticale, le film le plus fin en haut. L'épaisseur à laquelle les bandes se rejoignent indique la tendance à la coulure.

### - Evaluation de la viscosité

Elle s'effectue ici à l'aide d'un Brookfield^{®} RV à 25°C (mobile : S 4). La vitesse du mobile est fixée à 50 rpm et l'on mesure la viscosité de chaque peinture après que la viscosité soit stabilisée. On répète l'opération pour une vitesse de 20 rpm, 10 rpm, 5 rpm, 1 rpm.

### III - Préparation et caractérisation des organogélateurs, des additifs de rhéologie

### A) Préparation d'un mélange d'acides hydroxy- 9 et hydroxy-10 stéariques (9-HSA + 10-HSA)

La préparation est basée sur l'hydroxylation de la double liaison d'un acide gras (transposée ici à l'acide oléique) comme décrite dans « Addition of Formic acid to Oleifinic coumpounds » de HB Knights, R. E Koos and Daniel Swern, 2 Mai 1953.

D'autres méthodes peuvent être utilisées pour accéder aux acides gras monohydroxylés 9- et 10-HSA.

### Méthode de préparation utilisée

Dans un ballon de 1-Litre équipé d'un thermomètre, un Dean-Stark, un condensateur et d'un agitateur, on introduit sous une atmosphère d'azote, 319,1 grammes d'acide oléique, 677,6 grammes d'acide formique et 3,3 grammes d'acide perchlorique. Après 30 minutes à reflux, l'acide formique en excès est évaporé sous vide à 75 mbar et 65°C.

Le composé obtenu (102 grammes) est ensuite hydrolysé par une solution de soude 6N (100 grammes). Enfin, le produit est neutralisé par addition lente d'acide chlorhydrique fumant (64 grammes) dans 66 grammes d'eau.

La purification se fait par solubilisation du milieu réactionnel dans du toluène et par trois lavages successifs à l'aide d'une solution de NaCl 11%. Le toluène est ensuite évaporé et le produit est recristallisé dans l'hexane. 27 grammes d'un mélange d'acides gras monohydroxylés 9- et -10 HSA sont ainsi obtenus.

### B) Préparation de diamides selon l'invention et comparatifs

### EXEMPLE 1 : Diamide A comparatif

Dans un ballon de 1-Litre équipé d'un thermomètre, un Dean-Stark, un condensateur et d'un agitateur, on introduit dans une atmosphère d'azote, 49,96 grammes d'hexaméthylène diamine (soit 0,43 moles, 0,86 équivalent amine), 244,65 grammes (0,86 moles, 0,86 équivalents) d'acide stéarique.

Le mélange est chauffé à 200°C toujours sous courant d'azote. L'eau éliminée commence à s'accumuler dans le Dean Stark dès 150°C. La réaction est contrôlée par l'indice d'acide et d'amine. Lorsque les valeurs d'acides et d'amines sont inférieures à 10 mg KOH/g, le mélange réactionnel est refroidi à 150°C, puis déchargé dans un moule siliconé. Une fois refroidi à la température ambiante, le produit est micronisé mécaniquement par broyage et tamisage pour obtenir une granulométrie fine et contrôlée avec taille moyenne obtenue de 7 µm.

### EXEMPLE 2 : Diamide B selon l'invention

Dans un ballon de 1-Litre équipé d'un thermomètre, un Dean Stark, un condensateur et d'un agitateur, on introduit dans une atmosphère d'azote, 49,96 grammes d'hexaméthylène diamine (soit 0,43 moles, 0,86 équivalent amine), 260,48 grammes de mélange d'acides hydroxy-9 et -10 stéariques (soit 0,86 moles, 0,86 équivalent acide) comme décrit ci haut. Le mélange est chauffé à 200°C toujours sous courant d'azote. L'eau éliminée s'accumule dans le Dean Stark dès 150°C. La réaction est contrôlée par l'indice d'acide et d'amine. Lorsque les valeurs d'acide et d'amine sont inférieures à 10 mg KOH/g, le mélange réactionnel est refroidi à 150°C, puis déchargé dans un moule siliconé. Une fois refroidi à la température ambiante, le produit est micronisé mécaniquement comme dans l'exemple 1 avec même taille moyenne.

### EXEMPLES 3 et 4 : Diamide C comparatif (3) et diamide D selon l'invention (4)

Le même mode opératoire a été utilisé mais avec les composants de réaction suivants présentés dans le tableau ci-dessous :

**Tableau 2 : Diamides C et D**

| **Exemple** | **Réactif** | **Mole** | **Equivalents** |
|---|---|---|---|
| 3 Diamide C comparatif | L'éthylène diamine | 0,5 | 1 |
| | Acide Hexanoïque | 0,5 | 0,5 |
| | Acide stéarique | 0,5 | 0,5 |
| 4 Diamide D selon l'invention | L'éthylène diamine | 0,5 | 1 |
| | Acide Hexanoïque | 0,5 | 0,5 |
| | Acides 9 et 10 hydroxy-stéariques | 0,5 | 0,5 |

### EXEMPLES 5 et 6 : Diamide E comparatif (5) et diamide F selon l'invention (6)

Le même mode opératoire a été utilisé mais avec les composants de réaction suivants présentés dans le tableau ci-dessous :

**Tableau 3 : Diamides E et F**

| **Exemple** | **Réactif** | **Mole** | **Equivalents** |
|---|---|---|---|
| 5 Diamide E comparatif | L'éthylène diamine | 0,5 | 1 |
| | Acide Hexanoïque | 0,5 | 0,5 |
| | Acide 12- hydroxystéarique | 0,5 | 0,5 |
| 6 Diamide F selon l'invention | L'éthylène diamine | 0,5 | 1 |
| | Acide Hexanoïque | 0,5 | 0,5 |
| | Acides 9- et 10-hydroxy-stéariques | 0,25 | 0,25 |
| | Acide 12 hydroxystéarique | 0,25 | 0,25 |

### C) Propriété d'organogélateur pour diamides A et B

20 g de l'Exemple A-Comparatif préalablement broyé (taille moyenne en volume de 10 µ) et 80 g de xylène sont chargés dans une boite métallique à température ambiante. En utilisant un disperseur type Dispermat^{®} CV muni d'une pâle de 4 cm de diamètre, les deux produits sont mélangés à une vitesse de 2000 tours/min (ou rpm) pendant 30 minutes à une température n'excédant pas 20°C par régulation de la température par circulation d'eau froide.

Activation : La boite est ensuite refermée soigneusement et introduite dans une étuve préalablement préchauffée à 65°C pendant 24 heures. Une fois refroidi et après 4 heures de repos, le mélange reste liquide.

Suivant la même procédure, l'Exemple B selon l'invention se présente, après l'étape d'activation et de refroidissement, sous forme de pâte permettant à une spatule de bois de rester à la verticale dans la pâte. Ce résultat illustre parfaitement la capacité du diamide de l'Exemple B selon l'invention à être un organogélateur.

### IV - Evaluation des performances rhéologique dans une formulation de peinture Formulations de peintures utilisées pour l'évaluation

### 1 - Préparation

Une formulation dite « millbase » est préparée avec les proportions du tableau 3 de la manière suivante :
Dans un bol de disperseur (Dispermill 2075 yellow line, fournisseur : Erichsen^{®}) chauffé par un système de double enveloppe :
   1. Introduction des liants époxy ainsi que le dispersant et l'agent débullant. L'homogénéisation se fait après 2 minutes à 800 tours/minute.
   2. Introduction des charges et pigments puis broyage à 3000 tours minute pendant 30 minutes à l'aide d'une pâle de 7 cm. Grâce au bol à double enveloppe, cette étape est refroidie avec un bain d'eau froide (20°C).
   3. Introduction des solvants.

### 2 - Activation

24 heures après la préparation de la millbase (tableau 4), la formulation est de nouveau dispersée à 3000 tours par minute à l'aide d'une pâle de 4 cm. Les diamides C ou D sont introduits dans la millbase à une température d'activation donnée (variant de 40°C à 70°C) pendant 20 minutes à 3000 tours par minute.

Après l'ajout du durcisseur dilué (tableau 5) dans la millbase, les peintures sont ajustées avec un mélange xylène/butanol (1/1) à 0.4 Pa.s (mesuré sur le cône 4 à 25°C à 2500 s-1 à l'aide du Brookfield^{®} CAP 1000). Les proportions entre le durcisseur et le mélange de solvants sont définies dans le tableau 4.

Après l'ajustement, la peinture est mélangée à 1500 tours par minute durant 2 minutes, puis laissée au repos pendant 30 minutes.

**Tableau 4 : formulation « Millbase »**

| **Composition de la Millbase** | **Fonction** | **% massique** |
|---|---|---|
| Araldite^{®} GZ 7071X75 | liant | 17,3 |
| Araldite^{®} GY 783 BD | liant | 12,9 |
| BYK^{®} A530 | Agent débullant | 0,5 |
| Disperbyk^{®} 110 | Dispersant | 0,5 |
| Tiona 595 (Titanium dioxide) | Pigment | 1,9 |
| Bayferrox^{®} 915 595 (oxide de fer) | Pigment | 4,1 |
| ZP 10 (phosphate de zinc) | Pigment | 7,5 |
| Finntalc MO5 | charge | 9,4 |
| Silice HPF6 | charge | 19,0 |
| n-Butanol | Solvant | 5,4 |
| Diamide C ou D | Additif de Rhéologie | 0,8 |
| **TOTAL** | | **79,3** |

**Tableau 5 : durcisseur**

| **Composition du durcisseur** | **% massique** |
|---|---|
| CRAYAMID^{®} 140 | 8,8 |
| Xylène | 11,9 |
| **TOTAL** | **20,7** |

### 3 - Evaluation de la rhéologie des formulations et résultats

Différentes formulations de peinture ont été réalisées suivant les proportions du tableau 3 et 4 et avec différentes températures d'activation allant de 40 à 70°C suivant le protocole énoncé plus haut.

Les résultats de résistance à la coulure et de rhéologie montrent que le diamide D à base de 9- et 10-HSA a un effet thixotropique sur la formulation une fois qu'elle est activée à 60 ou 70°C contrairement au diamide C qui est inactif quelle que soit la température (tableau 7). Par ailleurs, le diamide D permet d'avoir une bonne résistance à la coulure (tableau 6). En conséquence, le diamide D à base de 9- et 10-HSA présente les caractéristiques nécessaires d'un additif de rhéologie.

**Tableau 6 : résultats de résistance à la coulure**

| **Essai** | **Diamide** | **Résistance à la coulure (µm)** |
|---|---|---|
| **Exemple 3, 40°C** | C | 225-250 |
| **Exemple 3, 50°C** | C | 225-250 |
| **Exemple 3, 60°C** | C | 225-250 |
| **Exemple 3, 70°C** | C | 225-250 |
| **Exemple 4, 40°C** | D | 225-250 |
| **Exemple 4, 50°C** | D | 225-250 |
| **Exemple 4, 60°C** | D | 375-400 |
| **Exemple 4, 70°C** | D | 400-425 |

**Tableau 7 : résultats rhéologiques**

| **Essai** | **Diamide** | **Viscosité Brookfield à 25°C (mPa.s)** | | | | |
|---|---|---|---|---|---|---|
| | | **1 RPM** | **5 RPM** | **10 RPM** | **50 RPM** | **100 RPM** |
| **Exemple 3, 40°C** | C | 2000 | 1150 | 906 | 657 | 588 |
| **Exemple 3, 50°C** | C | 1912 | 1002 | 858 | 642 | 580 |
| **Exemple 3, 60°C** | C | 1970 | 1055 | 880 | 640 | 584 |
| **Exemple 3, 70°C** | C | 1989 | 1107 | 900 | 654 | 588 |
| **Exemple 4, 40°C** | D | 2000 | 1120 | 920 | 660 | 596 |
| **Exemple 4, 50°C** | D | 2400 | 1280 | 1020 | 700 | 630 |
| **Exemple 4, 60°C** | D | 9600 | 3400 | 2300 | 1092 | 862 |
| **Exemple 4, 70°C** | D | 11800 | 4000 | 2640 | 1196 | 922 |

### V - Evaluation des performances rhéologique des diamides E (comparatif et F (selon l'invention) dans une formulation de peinture

Formulations de peintures utilisées pour l'évaluation : préparation et activation et évaluation, comme décrit pour amides C et D au point IV) avec mêmes formulations utilisées et testées sauf avec une activation à 60°C seulement.

### Résultats d'évaluation de la rhéologie des formulations

Les résultats de résistance à la coulure et de rhéologie montrent que le diamide F, à base de 9-HSA et 10-HSA et de 12 -HSA, a un effet thixotropique sur la formulation une fois qu'elle est activée à 60°C comme le diamide E à base de 12-HSA (voir tableau 9). Par ailleurs, le diamide F permet d'avoir une bonne résistance à la coulure (tableau 8). En conséquence, le diamide E à base de 9- et 10-HSA présente les caractéristiques nécessaires et satisfaisantes d'un additif de rhéologie.

**Tableau 8 : résultats de résistance à la coulure pour diamides E et F**

| **Essai** | **Diamide** | **Résistance à la coulure (µm)** |
|---|---|---|
| **Exemple 5, 60°C** | E | > 600 |
| **Exemple 6, 60°C** | F | > 600 |

**Tableau 9 : résultats rhéologiques**

| **Essai** | **Diamide** | **Viscosité Brookfield à 25°C** (**mPa**.**s**) | | | | |
|---|---|---|---|---|---|---|
| | | **1 RPM** | **5 RPM** | **10 RPM** | **50 RPM** | **100 RPM** |
| **Exemple 5, 60°C** | E | 22200 | 6760 | 4300 | 1804 | 1308 |
| **Exemple 6, 60°C** | F | 18800 | 8840 | 3720 | 1604 | 1186 |

## Revendications

1. Diamide d'acide gras, **caractérisé en ce qu'**il comprend au moins un produit de réaction d'un mélange comprenant ou consistant à :
a) au moins une diamine sélectionnée parmi :
▪ une diamine aliphatique linéaire en C₂ à C₁₂, et/ou
▪ une diamine cycloaliphatique en C₆ à C₁₈, et/ou
▪ une diamine aromatique
b) au moins un hydroxyacide carboxylique gras saturé sélectionné parmi l'acide hydroxy-9 stéarique et/ou l'acide hydroxy-10 stéarique, en présence ou en l'absence d'acide hydroxy-12 stéarique,
c) en option, au moins un monoacide sélectionné parmi les acides carboxyliques linéaires saturés et non hydroxylés en C₆ à C₁₈,
d) en option, au moins une deuxième diamine différente de a) et sélectionnée parmi les amines aliphatiques linéaires en C₂ à C₁₂.

2. Diamide d'acide gras selon la revendication 1, **caractérisé en ce que** ledit acide hydroxy-12 stéarique est présent et **en ce que** le taux des autres acides hydroxy stéariques, c'est-à-dire de l'acide hydroxy-9 stéarique et/ou de l'acide hydroxy-10 stéarique, varie de 10 à 99% en poids du composant b).

3. Diamide d'acide gras selon la revendication 1, **caractérisé en ce que** ledit acide hydroxy-12 stéarique est absent et qu'il est remplacé totalement, c'est-à-dire à 100%, par l'acide hydroxy-9 stéarique et/ou l'acide hydroxy-10 stéarique.

4. Diamide selon l'une des revendications 1 à 3, **caractérisé en ce que** ledit diamide porte 2 fonctions amides à base du même hydroxyacide b) ou à base de deux hydroxyacides b) différents.

5. Diamide selon l'une des revendications 1 à 3, **caractérisé en ce que** ledit monoacide c) est présent à un taux tel que le rapport molaire b/c dudit hydroxyacide b) sur ledit monoacide c) est de 1/2 à 4/1.

6. Diamide selon l'une des revendications 1 à 5, **caractérisé en ce qu'**il porte une fonction amide à base d'un hydroxyacide b) et une autre fonction amide à base dudit monoacide c) avec un rapport molaire b/c de 1/1.

7. Diamide selon l'une des revendications 1 à 6, **caractérisé en ce qu'**il comprend au moins 2 produits différents de réaction, tels que définis selon l'une desdites revendications 1 à 6.

8. Diamide selon la revendication 7, **caractérisé en ce qu'**il comprend un mélange de produits comprenant les produits de formules suivantes :
- b1-a1-c1
- b1-a1-b1
- b1-a1-b2
et de préférence :
- b1-a1-c1
- b1-a1-b1
- b1-a1-b2
- b2-a1-c1
- b2-a1-b2
avec a1 : résidu de diamine selon a), b1 : résidu de l'acide hydroxy-9 stéarique, b2 : résidu de l'acide hydroxy-10 stéarique et c1 : résidu de monoacide non hydroxylé selon monoacide c).

9. Diamide selon l'une des revendications 1 à 8, **caractérisé en ce qu'**il est sous forme de poudre micronisée.

10. Additif comprenant au moins un diamide tel que défini selon l'une des revendications 1 à 9, **caractérisé en ce qu'**il existe et est utilisé sous forme de pâte préactivée et préconcentrée dans un solvant organique.

11. Utilisation du diamide tel que défini selon l'une des revendications 1 à 9, **caractérisée en ce qu'**il est utilisé en tant qu'organogélateur dans un solvant organique pour l'obtention d'un organogel.

12. Utilisation selon la revendication 11, **caractérisée en ce que** ledit diamide est utilisé dans des compositions de revêtements, choisies parmi les peintures, vernis, encres, gel coats ou dans des compositions de colles ou d'adhésifs, de moulage, de mastics ou d'agents d'étanchéité ou d'agent décapant ou de cosmétique.

13. Organogélateur **caractérisé en ce qu'**il comprend au moins un diamide tel que défini selon l'une des revendications 1 à 9.

14. Organogélateur selon la revendication 13, **caractérisé en ce qu'**il s'agit d'un agent ou d'additif de rhéologie dans une composition préconcentrée dans un solvant organique sous forme de pâte préactivée.

15. Composition de liant organique, **caractérisée en ce qu'**elle comprend en tant qu'agent de rhéologie au moins un diamide tel que défini selon l'une des revendications 1 à 9 ou un additif tel que défini selon la revendication 10.

16. Composition de liant organique selon la revendication 15, **caractérisée en ce que** ledit liant organique est un liant pour compositions de revêtements parmi peintures, vernis, encres, gel coats ou un liant pour compositions de colles ou d'adhésifs ou un liant pour compositions de mastic ou d'agents d'étanchéité ou d'agent décapant ou pour des compositions de moulage ou pour des compositions de cosmétique.

17. Composition selon la revendication 15 ou 16, **caractérisée en ce que** ledit liant est sélectionné parmi : résines époxy, polyesters insaturés et saturés, vinyl esters, alkydes, résines silanées, polyuréthanes, polyesters-amides, résines acryliques solvantées avec solvant non réactif, monomères et/ou oligomères acryliques multifonctionnels ou résines acryliques acrylées avec diluant réactif ou élastomères chlorés ou non chlorés ou autres polymères chlorés.

## Patentansprüche

1. Fettsäurediamid, **dadurch gekennzeichnet, dass** es mindestens ein Reaktionsprodukt eines Gemischs umfasst, das Folgendes umfasst oder daraus besteht:
a) mindestens ein Diamin, ausgewählt aus:
▪ einem geraden aliphatischen C₂- bis C₁₂-Diamin und/oder
▪ einem cycloaliphatischen C₆- bis C₁₈-Diamin und/oder
▪ einem aromatischen Diamin,
b) mindestens eine gesättigte Hydroxyfettcarbonsäure, ausgewählt aus 9-Hydroxystearinsäure und/oder 10-Hydroxystearinsäure bei Vorliegen oder Fehlen von 12-Hydroxystearinsäure,
c) gegebenenfalls mindestens eine Monosäure, ausgewählt aus gesättigten, geraden und nicht-hydroxylierten C₆- bis C₁₈-Carbonsäuren,
d) gegebenenfalls mindestens ein zweites Diamin, das von a) verschieden und aus geraden aliphatischen C₂- bis C₁₂-Aminen ausgewählt ist.

2. Fettsäurediamid nach Anspruch 1, **dadurch gekennzeichnet, dass** 12-Hydroxystearinsäure vorhanden ist und dass der Anteil der anderen Hydroxystearinsäuren, d. h. 9-Hydroxystearinsäure und/oder 10-Hydroxystearinsäure, von 10 bis 99 Gew.-%, bezogen auf das Gewicht der Komponente b), variiert.

3. Fettsäurediamid nach Anspruch 1, **dadurch gekennzeichnet, dass** 12-Hydroxystearinsäure fehlt und dass sie vollständig, d. h. zu 100%, durch 9-Hydroxystearinsäure und/oder 10-Hydroxystearinsäure ersetzt ist.

4. Diamid nach einem der Ansprüche 1 bis 3, **dadurch gekennzeichnet, dass** das Diamid zwei Amidfunktionen auf der Basis der gleichen Hydroxysäure b) oder auf der Basis von zwei verschiedenen Hydroxysäuren b) trägt.

5. Diamid nach einem der Ansprüche 1 bis 3, **dadurch gekennzeichnet, dass** die Monosäure c) in einem derartigen Anteil vorliegt, dass das Molverhältnis b/c der Hydroxysäure b) zu der Monosäure c) 1/2 bis 4/1 beträgt.

6. Diamid nach einem der Ansprüche 1 bis 5, **dadurch gekennzeichnet, dass** es eine Amidfunktion auf der Basis einer Hydroxysäure b) und eine andere Amidfunktion auf der Basis der Monosäure c) bei einem Molverhältnis b/c von 1/1 trägt.

7. Diamid nach einem der Ansprüche 1 bis 6, **dadurch gekennzeichnet, dass** es mindestens 2 verschiedene Reaktionsprodukte nach einem der Ansprüche 1 bis 6 enthält.

8. Diamid nach Anspruch 7, **dadurch gekennzeichnet, dass** es ein Produktegemisch umfasst, das die Produkte der folgenden Formeln umfasst:
- b1-a1-c1
- b1-a1-b1
- b1-a1-b2
und vorzugsweise:
- b1-a1-c1
- b1-a1-b1
- b1-a1-b2
- b2-a1-c1
- b2-a1-b2
wobei a1: Diamin-Rest gemäß a), b1: 9-Hydroxystearinsäure-Rest , b2: 10-Hydroxystearinsäure-Rest und c1: Rest der nicht-hydroxylierten Monosäure gemäß der Monosäure c).

9. Diamid nach einem der Ansprüche 1 bis 8, **dadurch gekennzeichnet, dass** es in Form von mikronisiertem Pulver vorliegt.

10. Additiv, das mindestens ein Diamid nach einem der Ansprüche 1 bis 9 umfasst, **dadurch gekennzeichnet, dass** es in Form einer voraktivierten und vorkonzentrierten Paste in einem organischen Lösungsmittel vorliegt und verwendet wird.

11. Verwendung des Diamids nach einem der Ansprüche 1 bis 9, **dadurch gekennzeichnet, dass** es als Organogeliermittel in einem organischen Lösungsmittel zum Erhalten eines Organogels verwendet wird.

12. Verwendung nach Anspruch 11, **dadurch gekennzeichnet, dass** das Diamid in Beschichtungszusammensetzungen, ausgewählt aus Anstrichen, Lacken, Tinten, Gelüberzügen, oder in Leim- oder Klebstoff-, Guss-, Dichtmassen- oder Dichtungsmittel- oder Abbeizmittel- oder Kosmetikzusammensetzungen verwendet wird.

13. Organogeliermittel, **dadurch gekennzeichnet, dass** es mindestens einem Diamid nach einem der Ansprüche 1 bis 9 umfasst.

14. Organogeliermittel nach Anspruch 13, **dadurch gekennzeichnet, dass** es sich um ein Rheologiemittel oder -additiv in einer vorkonzentrierten Zusammensetzung in einem organischen Lösungsmittel in Form einer voraktivierten Paste handelt.

15. Zusammensetzung eines organischen Bindemittels, **dadurch gekennzeichnet, dass** sie als Rheologiemittel mindestens ein Diamid nach einem der Ansprüche 1 bis 9 oder ein Additiv nach Anspruch 10 umfasst.

16. Zusammensetzung eines organischen Bindemittels nach Anspruch 15, **dadurch gekennzeichnet, dass** das organische Bindemittel ein Bindemittel für Beschichtungszusammensetzungen aus Anstrichen, Lacken, Tinten, Gelüberzügen, oder ein Bindemittel für Leim- oder Klebstoffzusammensetzungen oder ein Bindemittel für Dichtmassen- oder Dichtungsmittel- oder Abbeizmittelzusammensetzungen oder für Gusszusammensetzungen oder für Kosmetikzusammensetzungen ist.

17. Zusammensetzung nach Anspruch 15 oder 16, **dadurch gekennzeichnet, dass** das Bindemittel aus den Folgenden ausgewählt wird: Epoxidharzen, ungesättigten und gesättigten Polyestern, Vinylestern, Alkyden, Silanharzen, Polyurethanen, Polyesteramiden, lösungsmittelhaltigen Acrylharzen mit nicht-reaktivem Lösungsmittel, multifunktionellen Acrylmonomeren und/oder -oligomeren oder acrylierten Acrylharzen mit reaktivem Verdünnungsmittel oder chlorierten oder nicht chlorierten Elastomeren oder anderen chlorierten Polymeren.

## Claims

1. Fatty acid diamide, **characterized in that** it comprises at least one product of reaction of a mixture comprising or consisting of:
a) at least one diamine selected from:
▪ a linear aliphatic C₂ to C₁₂ diamine, and/or
▪ a C₆ to C₁₈ cycloaliphatic diamine, and/or
▪ an aromatic diamine,
b) at least one saturated hydroxycarboxylic fatty acid selected from 9-hydroxystearic acid and/or 10-hydroxystearic acid in the presence or absence of 12-hydroxystearic acid,
c) optionally, at least one monoacid selected from saturated, non-hydroxylated C₆ to C₁₈ linear carboxylic acids,
d) optionally, at least a second diamine different from a) and selected from linear aliphatic C₂ to C₁₂ amines.

2. Fatty acid diamide according to Claim 1, **characterized in that** said 12-hydroxystearic acid is present and **in that** the content of the other hydroxystearic acids, i.e. 9-hydroxystearic acid and/or 10-hydroxystearic acid, ranges from 10% to 99% by weight of component b).

3. Fatty acid diamide according to Claim 1, **characterized in that** said 12-hydroxystearic acid is absent and **in that** it is totally replaced, i.e. to 100%, with 9-hydroxystearic acid and/or 10-hydroxystearic acid.

4. Diamide according to one of Claims 1 to 3, **characterized in that** said diamide bears two amide functions based on the same hydroxy acid b) or based on two different hydroxy acids b).

5. Diamide according to one of Claims 1 to 3, **characterized in that** said monoacid c) is present in a content such that the b/c mole ratio of said hydroxy acid b) to said monoacid c) is from 1/2 to 4/1.

6. Diamide according to one of Claims 1 to 5, **characterized in that** it bears one amide function based on a hydroxy acid b) and another amide function based on said monoacid c) with a b/c mole ratio of 1/1.

7. Diamide according to one of Claims 1 to 6, **characterized in that** it comprises at least two different reaction products, as defined according to one of said Claims 1 to 6.

8. Diamide according to Claim 7, **characterized in that** it comprises a mixture of products comprising the products having the following formulae:
- b1-a1-c1
- b1-a1-b1
- b1-a1-b2
and preferably:
- b1-a1-c1
- b1-a1-b1
- b1-a1-b2
- b2-a1-c1
- b2-a1-b2
with a1: diamine residue according to a), b1: 9-hydroxystearic acid residue, b2: 10-hydroxystearic acid residue and c1: non-hydroxylated monoacid residue according to monoacid c).

9. Diamide according to one of Claims 1 to 8, **characterized in that** it is in the form of a micronized powder.

10. Additive comprising at least one diamide as defined according to one of Claims 1 to 9, **characterized in that** it exists and is used in the form of a paste preactivated and preconcentrated in an organic solvent.

11. Use of the diamide as defined according to one of Claims 1 to 9, **characterized in that** it is used as an organogelator in an organic solvent for producing an organogel.

12. Use according to Claim 11, **characterized in that** said diamide is used in coating compositions, chosen from the paints, varnishes, inks and gel coats, or in glue or adhesive, molding, mastic or sealing or stripping or cosmetic compositions.

13. Organogelator, **characterized in that** it comprises at least one diamide as defined according to one of Claims 1 to 9.

14. Organogelator according to Claim 13, **characterized in that** it is a rheology agent or additive in a composition preconcentrated in an organic solvent in the form of a preactivated paste.

15. Organic binder composition, **characterized in that** it comprises as rheology agent at least one diamide as defined according to one of Claims 1 to 9 or an additive as defined according to Claim 10.

16. Organic binder composition according to Claim 15, **characterized in that** said organic binder is a binder for coating compositions chosen from paints, varnishes, inks and gel coats, or a binder for glue or adhesive compositions or a binder for mastic or sealing or stripping compositions or for molding compositions or for cosmetic compositions.

17. Composition according to Claim 15 or 16, **characterized in that** said binder is selected from: epoxy resins, unsaturated and saturated polyesters, vinyl esters, alkyds, silanized resins, polyurethanes, polyesteramides, solvent-based acrylic resins with an unreactive solvent, multifunctional acrylic monomers and/or oligomers or acrylated acrylic resins with reactive diluent or chlorinated or non-chlorinated elastomers or other chlorinated polymers.
